# EUROPEAN PATENT SPECIFICATION

(11) **EP 0 197 797 B1**
(45) Date of publication and mention of the grant of the patent: **20.10.1993**
(21) Application number: 86302617.5
(22) Date of filing: 09.04.1986
(51) Int. Cl.: C12N 15/26, C12P 21/02, C12N 1/20, C12N 15/63, C12N 15/71

(54) **Improved recombinant hosts, their production and use**
Rekombinante Wirte, deren Herstellung und Verwendung
Hôtes recombinants, leur production et leur utilisation

(30) Priority: 10.04.1985 US 721794
(43) Date of publication of application: 15.10.1986
(73) Proprietor: CETUS ONCOLOGY CORPORATION, Emeryville California 94608 (US)
(72) Inventor: Gelfand, David Harrow, Oakland California 94611 (US); Bauer, Keith Alan, Oakland California 94610 (US)
(74) Representative: Bizley, Richard Edward

(56) References cited:
- EP-A- 0 052 002
- AU-A- 549 686
- US-A- 4 371 614
- METHODS IN ENZYMOLOGY, vol. 101, 1983, pages 155-164, Academic Press, Inc.; B.P. NICHOLS et al.: "Plasmids containing the trp promoters of Escherichia coli and Serratia marcescens and their use in expressing cloned genes"
- PROC. NATL. ACAD. SCI., vol. 79, May 1982, pages 3120-3124; R.L. KELLEY et al.: "Trp aporepressor production is controlled by autogenous regulation and inefficient translation"
- PROC. NATL. ACAD. SCI, vol. 82, January 1985, pages 483-487; R.L. KELLEY et al.: "Multational studies with the trp repressor of Escherichia coli support the helix-turn-helix model of repressor recognition of operator DNA"
- GENE, vol. 9, 1980, pages 27-47, Elsevier/North-Holland Biomedical Press; R.A. HALLEWELL et al.: "Plasmid vectors containing the tryptophan operon promoter suitable for efficient regulated expression of foreign genes"
- NUCLEIC ACIDS RESEARCH, vol. 9, 1981, pages 6647-6668, IRL Press Limited, London, GB; C. YANOFSKY et al.: "The complete nucleotide sequence of the tryptophan operon of Escherichia coli"
- C. Yanofsky "De la Physique théorique à la Biologie" comptes rendus de la seconde Conférence internationale de Physique Théorique et Biologie (M. Marois, ed. p. 191-205, CNRS, Paris 1971

## Description

The invention relates to the recombinant production of heterologous proteins under regulatable promoter control. More specifically, the invention relates to providing hosts with at least one modification in the genome associated with aporepressor-activator production. The presence of the modification permits more effective production of the desired protein.

Feedback control systems which regulate the metabolic pathways of both procaryotic and eucaryotic organisms have been known for many years to exist both at the genetic and protein levels. Perhaps the best known examples of genetic level feedback control are regulatable promoter/operator systems, such as the β-galactosidase (lac) promoter/operator and the tryptophan (trp) promoter/operator systems in procaryotic hosts. The lac operator responds to a constitutive protein repressor to prevent transcription. In the presence of an inducer such as lactose or IPTG, the repressor is bound to inducer, and unable to bind to the operator, thus transcription is permitted. The trp operator, conversely, responds only to an "activated" repressor protein: i.e., in the absence of tryptophan, the repressor does not bind to operator. Advantage has been taken of these genetic level controls to effect the production of recombinant proteins at times of the host cells' life cycle which are advantageous to efficient production.

Typically, the coding sequence for a desired heterologous protein is ligated in operable configuration with the regulatable promoter/operator in suitable vectors to transform the host. Since immediate production of the heterologous protein during the growth phase of the transformed organism would be injurious to the healthy multiplication of the bacteria, this production is delayed by causing production of sufficient repressor to keep the promoter/operator system turned off.

In the case of the trp operator, the mediator of the on/off switch is the combination of tryptophan with a repressor protein (aporepressor) produced normally by the host, but at a level dependent on the concentration of tryptophan in the medium. Combination with tryptophan permits the aporepressor to function, and expression of coding sequences under control of the trp promoter is delayed by the presence of tryptophan until further repression is no longer needed. Under usual recombinant host growth conditions, derepression is effected either by depleting the supply of tryptophan in the medium or by supplying an antagonist to tryptophan such as indole acrylic acid (IAA), which interferes with the ability of tryptophan to bond to the aporepressor.

While, therefore, in principle, the trp promoter system offers a convenient means to delay expression of heterologous gene sequences until a desired point in the growth curve is reached and then permit operation of the promoter, this control is not absolute. One difficulty is that complete depletion of the tryptophan is impractical because tryptophan is a component of many proteins, and is required at some level to enable protein synthesis. The result is that the trp promoter is always partially repressed due to this minimal tryptophan level.

The mechanisms for regulating tryptophan production have been studied by C. Yanofsky and his colleagues for many years. Studies conducted more than twenty years ago provided a revertant of a tryptophan-requiring mutant to partial tryptophan independence. This partial revertant apparently exhibited higher expression levels of the gene sequences under trp promoter control, but exhibited a slower growth rate without tryptophan than does wild type. A study of this mutant, A46PR9, (Henning, U., et al., Proc Natl Acad Sci (USA) (1962) 48:1497-1504), showed that a mutation in the coding sequence for trpA, an enzyme which catalyzes one of the steps in the conversion of indole glycerol phosphate to tryptophan, contains a mutation which results in the substitution of a valine residue for a glycine residue. The result is a protein which is less active in performing this catalysis than the native protein, thus accounting for the slow rate of growth in the absence of tryptophan. On the other hand, higher level amounts of this relatively inactive protein are formed, thus indicating a higher degree of expression for the genomic sequences under control of the trp promoter/operator.

The importance of this trp operon regulation for enhanced expression is further elucidated in a more recent paper of the same group (Kelley, R. L., et al., Proc Natl Acad Sci (USA) (1982), 79:3120-3124). LacZ fusion flags were used to show that the response of a typical procaryotic cell to variations in tryptophan concentrations is regulated mainly by the trp operon, which shows a 70-fold variation over the tryptophan concentration range studied, whereas the trp aporepressor synthesis showed a variation of only 4-5 fold.

Nichols, B.P. et al, in Methods in Enz., Vol. 101, pp. 155-164, describe the use of the E.coli and S.marcescens trp promoters in expression plasmids to achieve the over production of trp operon polypeptides. In addition, mention is made of the use of the E.coli trp promoter to drive the expression of a variety of polypeptides. The induction of expression in this system may be accomplished by various means. Constitutive expression is achieved in strains lacking the trp aporepressor gene (trpR). Strains having a temperature-sensitive trp aporepressor are induced by temperature shift while strains containing functional trp aporepressor may be induced by creating mild tryptophan starvation conditions. One way to do this is by the addition of a tryptophan analogue to the medium and another is to use a strain having a mutation in one of the trp structural genes such that tryptophan biosynthesis is inefficient and starvation occurs in minimal medium. However, the choice of the induction method should be influenced by the tryptophan content of the protein to be overproduced. It is noted that trp expression plasmids lacking the trp leader-attenuator control region are notoriously unstable when induced.

Kelly, R.L. et al, in Proc.Natl.Acad.Sci., Vol 82, January 1985, pp. 483-487 sought to analyse the interaction of the trp repressor protein with the operator DNA sequences of the three operons it regulates. This is done using trp repressor mutants and determining the amino acid substitutions which affect DNA binding without affecting dimerization of the repressor. The conclusion is that the mutations reported are consistent with the hypothesis that repressors recognise specific operator sequences via a helix-turn-helix structure.

Since, therefore, the level of production of heterologous proteins under trp control in recombinant hosts depends on the ability of the trp promoter to exhibit a high level of efficiency in promoting transcription, it would be desirable to introduce, in recombinant hosts, alterations which would result in more dramatic derepression. From results with A46PR9 it would appear that the coding sequence for the modified trpA protein results in enhanced expression levels effected by the trp promoter operably linked to its own coding sequence. The effect could be applicable with regard to any trp promoter in the cell, including a trp promoter in operable linkage with the recombinant heterologous coding sequences. Therefore, hosts having this alteration in the genome may be high producers when transformed with vectors using trp control.

Analogous host cell modifications affecting promoters susceptible to similar feedback control mechanisms are also desirable in instances where these promoters can be used to control expression of heterologous proteins. For example, the phenylalanine A (pheA) promoter/operator and tyrosine (aroF) promoter/operator are less frequently used for recombinant expression. However, their sequences and mode of regulation are known, and their use for the production of heterologous proteins is within the skill of the art. (See Hudson, G. S., et al., J Mol Biol (1984) 180:1023-1051.)

Such host cell improvements are the subject of the present invention.

Thus, the invention provides a recombinant expression system for heterologous protein which comprises:
a procaryotic host having an operon controlled by an indigenous regulatable promoter, which host is transformed with a DNA sequence containing a desired heterologous coding sequence in operable linkage to the same regulatable promoter,
wherein said host incorporates a genomic alternation which permits increased expression (as compared to wild type) of said heterologous protein and wherein the heterologous protein is a protein not involved in the biosynthesis of tryptophan.

Hosts in the present invention may be prepared by altering the genome of the host organism to obtain mutant sequences under control of the same promoter as that used for the expression of the recombinant DNA sequence, which mutant sequences result in increased levels of their own expression. Typically the mutant sequences encode enzymes responsible for the synthesis of an aporepressor-activator. The result is that derepression of expression is enhanced.

Thus, in one aspect, the invention is directed to a recombinant host cell with diminished ability to synthesize an aporepressor-activator. This host cell has an alteration in its genome resulting in enhanced expression under the control of a promoter which can also be used to control recombinant protein production in the same host. Another aspect of the invention is a protein expression system comprising the foregoing host transformed with a vector containing the coding sequence for a desired protein under the control of a promoter/operator which is responsive to the same aporepressor-aporepressor-activator as is the operon which is modified so as to be more effectively derepressed.

### Brief Description of the Drawings

Figure 1 shows a diagram of the process for KB2 production using P1 phage containing cysB⁻, Tn5 and P1 phage containing trpA46PR9.

Figure 2 shows the construction of the expression vector for IL-2, pLW45.

Figure 3 shows stained SDS-gels run on extracts from pLW45 transformed hosts.

### Modes of Carrying Out the Invention

### A. Definitions

As used herein, "aporepressor-activator" refers to a substance which combines with an aporepressor protein. The combination is effective in inhibiting expression under the control of a target promoter.

In a commonly encountered form of transcription control, the promoter is provided with an operator sequence, which, when bound with a suitable repressor, shuts off the transcription under the subject promoter's control. The repressor may be a protein which is produced under the control of an entirely different promoter elsewhere in the cell's DNA which is, by itself, capable of combining with the operator sequence to shut off the subject gene. This is the situation with respect to the lac (β-galactosidase) promoter/operator, which is controlled by a repressor protein produced under the control of a different promoter. However, a number of other control systems require that the aporepressor protein be activated by combination with another substance--the "aporepressor-activator". Typically this substance is the end product of the synthetic sequence whose production is controlled by the subject gene. Familiar examples are the trp promoter, where the aporepressor (trpR) must be combined with tryptophan in order to repress; tyrosine (aroF) promoter, which controls the synthesis of enzymes in the pathway to tyrosine, which is regulated by an aporepressor (tyrR) in combination with tyrosine; and the analogous operon controlling the production of enzymes required in the synthesis of phenylalanine where the aporepressor also requires combination with the end product to be effective. Thus, "aporepressor-activator" refers to those substances which must be available to combine with the aporepressor protein in order to control the subject regulatable promoter.

"Repressor" refers to a substance effective in this regard; it may be an aporepressor (protein) alone or sold protein in combination with the aporepressor-activator.

"Regulatable promoter" refers to a promoter whose operation is controlled by the presence or absence of a substance in a cell or cell culture such as a repressor, or by other environmental conditions such as temperature, salt concentration, and so forth.

"Operably linked" refers to juxtaposition wherein the functionality of the operably linked subjects are preserved. Thus, promoter operably linked to a coding sequence may result in expression of the coding sequence under control of the promoter under proper conditions.

"Cells", "cell cultures", "host cells", "recombinant host cells" refer to subject cells for recombinant DNA manipulations. As would be apparent from the context, these cells may be candidates for, or resultants of, transfer of new DNA sequences according to recombinant techniques. Techniques which are suitable for DNA uptake by cells include, most prominently, in vitro transformation; however, other techniques such as transduction or conjugation may also be used. The definition further includes the progeny of the cells directly referred to. It is understood that such progeny may not be precisely identical in DNA content to their parents, but such progeny are included in the definition so long as alterations due, for example, to accidental or deliberate mutation do not destroy the ability of the cells to exhibit the properties conferred by the DNA introduced in a manner similar to that exhibited by their parents.

### B. General Description

Any mutant host, produced by whatever means, having a genomic modification which results in increased expression (as compared to wild type) of sequences under control of a regulatable promoter when the promoter is derepressed is a candidate host for the expression systems of the invention. For example, in the illustration below, E. coli trp A46PR9 could, itself, be used as a host. If the organism has, however, other characteristics which make it less desirable as a recombinant host, it may nevertheless be used as a donor system for the genomic modification as is the case for E. coli trp A46PR9 in the illustration below. For example, a standard recombinant production strain of E. coil can be modified to contain mutations in its genome which increase the level of expression for an aporepressor activator operon by first selecting these suitable mutants of a donor strain, and then using a standard phage transduction technique to introduce the desired mutation. In the alternative, the production strain may be subjected to mutation pressure through irradiation or chemical treatment, and desirable mutants selected. The host, whose genomic sequences have been altered in the aporepressor activator operon, may then be used to produce recombinant proteins by introducing a vector which contains an expression system for the desired recombinant protein under the control of the same regulatable promoter as that controlling the aporepressor-activator operon.

While E. coli hosts are exemplified below, and are clearly the most convenient for routine production, other forms of procaryotic hosts are clearly not excluded from the invention. Therefore, strains of bacillus, for example, Bacillus subtilis or of Pseudomonas, might also be used as subjects for constructing the host organisms useful in the invention. Similarly, while the trp promoter/operator and trp operon are exemplified, any operon system which is controlled analogously could be used. Thus, for example, the operons controlling production of tyrosine or phenylalanine may also be used.

In general, in a convenient method to prepare the host, the desired substrate host is first infected with general transducing phage stock obtained from lysis of an infected corresponding bacterium containing a marker mutation which maps on the genome proximal to the locus of the desired change. The successfully transduced hosts, now containing the marker, are than transduced with a general transducing phage stock which has been obtained by lysis of cells which contain the desired mutation. The disappearance of the marker can then be used to select for transductants with a high probability of integration of genomic sequences containing the desired mutation. The resulting desired transductants are then suitable as hosts for the recombinant sequences containing the corresponding regulatable promoters.

### C. Examples

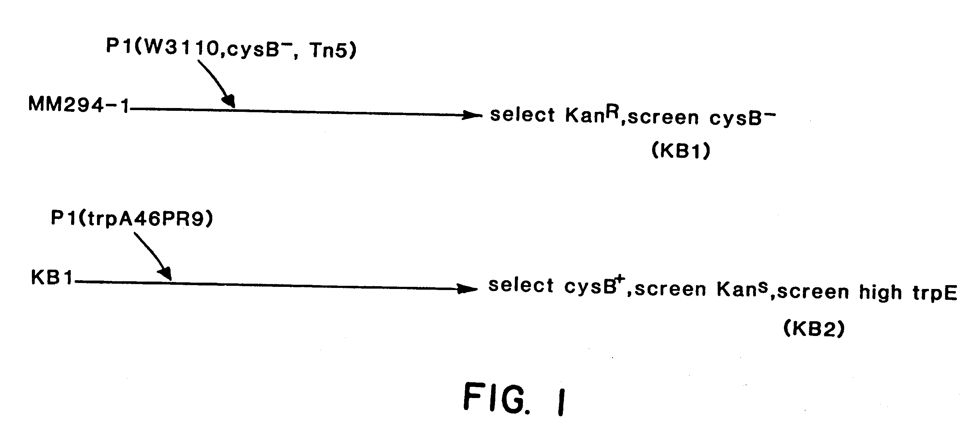

The following example is intended to illustrate, but not to limit, the invention. The example uses an alteration of the trp operon which is transduced into a convenient E. coli recombinant host strain by the aforementioned phage transduction techniques. The construction of the host KB2 is shown in Figure 1.

E. coli strain W3110 cysB⁻, Tn5 is used as a donor strain for the marker sequences. The cysB⁻ locus is at approximately 28.0' on the E. coli genome, which is proximally adjacent to the trp operon locus at 27.7'. The Tn5 transposon in the donor strain is proximal to the cysB⁻ also, and the presence of Tn5 results in resistance to the antibiotics kanamycin, neomycin, and other aminoglycosides. The presence of the closely linked Tn5 permits selection of transductants using resistance to these antibiotics.

Accordingly, E. coli W3110 (cysB⁻, Tn5) is infected with the standard transducing phage P1 and the resulting lysates used to transduce the recombinant production strain E. coli K12 MM294-1. The transduction is performed essentially as described by Lennox, E. S., Virology (1955) 1:190, as modified by Yanofsky, et al., Virology (1959) 8:425, and Maling, R. D., et al., Proc Natl Acad Sci (Wash) (1964) 47:551.

The transduced cells were then plated on media containing 30 µg/ml kanamycin to select for Kan^{R}. Successful colonies were screened for cysB⁻, i.e., a growth requirement for cysteine, and approximately 20% of the kanamycin-resistant (Kan^{R}) colonies showed this characteristic. One such colony, designated KB1, was selected as the substrate host.

E. coli KB1 was then transduced using lysates of P1-infected E. coli trpA46PR9, obtained from Professor Yanofsky at Stanford University, a strain generally available upon request. This donor strain is wild type with respect to cysB. The transductant cultures obtained from KB1 using these lysates were then selected for cysB⁺ by growth in medium lacking cysteine. The selected cultures were screened for kanamycin sensitivity, and approximately 50% of the transductants were kanamycin sensitive. Finally, the kanamycin-sensitive colonies were screened for the presence of the desired mutation by assessing the level of anthranilate synthase (trpE gene product) another enzyme besides tryptophan synthetase (trpA gene product) produced by the trp operon--in colonies grown on glucose-minimal media. Eleven percent of the kanamycin-sensitive colonies showed high levels of this enzyme, and accordingly were apparently more efficient than wild type in producing the protein products of the trp operon.

The resulting successful strain, designated E. coli MM294/A46PR9 KB2, was deposited with ATCC on March 29, 1985 under the provisions of the Budapest Treaty and given accession number 53,075.

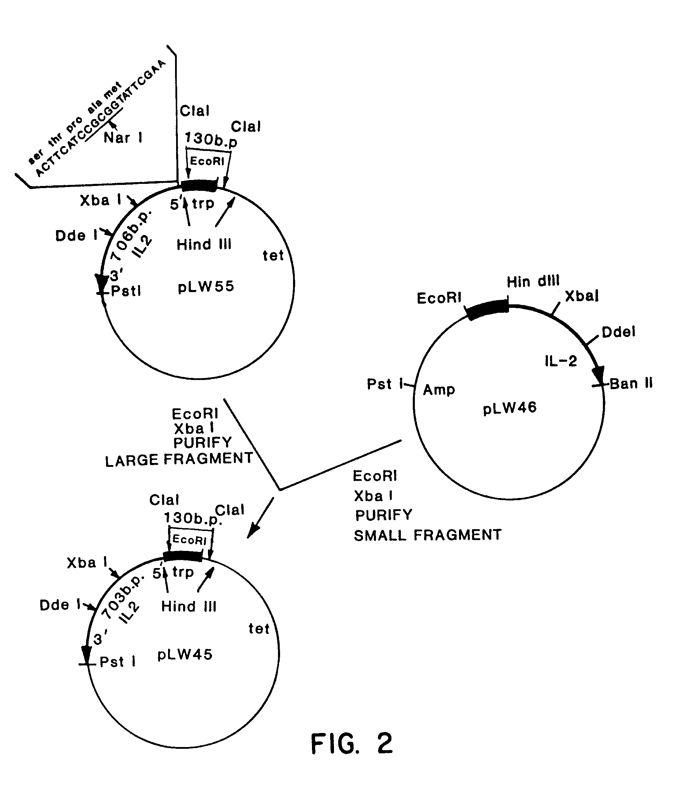

E. coli KB2 was transformed using the standard transformation technique for procaryotes of Cohen, S.N. et al., Proc Natl Acad Sci (1972) 69:2110 with pLW45. pLW45, shown in Figure 2, encodes an Interleukin 2 (IL-2) mutein having the cysteine residue at position 125 replaced by a serine. The IL2-coding sequences in pLW45 are under the control of the trp promoter, and the vector confers tetracycline resistance. The pLW45 plasmid may be prepared from two analogous plasmids described in U.S. Patent No. 4,518,584, granted May 21, 1984, assigned to the same assignee and incorporated herein by reference. These plasmids, pLW55 and pLW46, have been deposited with the ATCC and have accession nos. 39,516 and 39,452, respectively, and are now available to the public under the provisions of the Budapest Treaty.

pLW45 is identical to pLW55, except that the N-terminal sequence of the (pLW45) IL-2 lacks an alanine codon of the native sequence. pLW46 contains the desired N-terminal sequence in the same operable linkage with the trp promoter as is found in pLW55. Accordingly, pLW45 is constructed by excising an EcoRI/XbaI fragment from pLW46 (the fragment containing the trp promoter and the N-terminal portion of IL-2) and inserting this fragment into EcoRI/XbaI-digested pLW55.

The transformed hosts were grown in a fermenter containing the following medium:

| | |
|---|---|
| (NH₄)₂SO₄ | 150 mM |
| KH₂PO₄ | 21.6 mM |
| Na₃ Citrate | 1.5 mM |
| ZnSO₄ · 7H₂O | 60 µM |
| MnSO₄ · H₂O | 60 µM |
| CuSO₄ · 5H₂O | 2 µM |
| pH adjusted to 6.50 with 2.5 N NaOH autoclaved. | |

### Sterile Additions (post autoclave)

| | |
|---|---|
| MgSO₄ · 7H₂O | 3 mM |
| FeSO₄ · 7H₂O | 100 µM |
| L-tryptophan | 70 mg/l |
| Thiamine-HCl | 20 mg/l |
| Glucose | 5 g/l |
| Tetracycline | 5 mg/l |

Polypropyene glycol antifoam, glucose, 50% solution, and KOH, 5 N, were added on demand.

The pH of the fermenter was maintained at 6.8 with 5 N KOH. Residual glucose was maintained between 5-10 g/l, dissolved oxygen at 40%, and temperature at 37°C. Harvest was made four hours after the OD₆₈₀ reached about 10. Previous results indicate that tryptophan is depleted and induction occurs at approximately OD₆₈₀=10.

(In other fermenter runs, casamino acids were added to 2% at approximately OD₆₈₀=10, at the start of induction. Under these conditions, the MM294-1 controls produced IL-2 mutein at somewhat higher level (8%-9%) than those indicated below. This additional step was not necessary or helpful in fermentation using MM294/A46PR9.)

Samples of the fermenter culture were removed periodically during the fermentation. Samples were prepared for SDS-PAGE by boiling in SDS, and then applied to gels. The developed gels from the resulting crude extracts were stained with Coomassie blue and scanned with a densitometer. Estimation was thereby made of the quantity of IL-2 mutein produced both in control cultures using MM294-1 as host and pLW45 as transforming plasmid and in the extracts from pLW45 transformed hosts of the invention. While the induced control organisms produced 3%-5% of total cell protein as IL-2 mutein, the induced transformed cells of the invention produced IL-2 mutein at a level of 18%-20% of total cell protein.

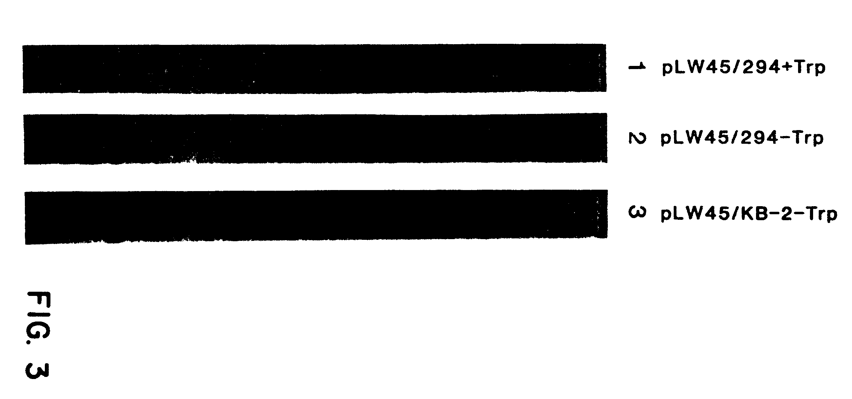

These results are shown in Figure 3. Lane 1 shows proteins from MM294-1 transformed with pLW45 extracted prior to induction; lane 2 shows proteins from these cells after induction; lane 3 shows proteins extracted from induced pLW45-transformed E. coli MM294/A46PR9 (KB2).

The recombinant host system of the present invention may be used in the expression of a wide variety of heterologous proteins or polypeptides. In this connection, the recombinant host system may be used to express proteins or polypeptides such as interferons (alpha, beta, or gamma), lymphokines (interleukins 1, 2 or 3, colony stimulating factors G, GM or 1, tumor necrosis factor) lymphotoxins, leukoregulin, hormones (e.g., insulin), vaccines, enzymes, growth factors, and muteins of these proteins and polypeptides.

## Claims

1. A recombinant expression system for heterologous proteins not involved in the biosynthesis of tryptophan which comprises:
a procaryotic host having an operon controlled by an indigenous regulatable promoter, which host is transformed with a DNA sequence containing a desired heterologous coding sequence in operable linkage to the regulatable promoter,
wherein the operon incorporates a mutation in a sequence encoding an enzyme responsible for the synthesis of aporepressor-activator resulting in diminished ability to synthesize the aporepressor-activator, which permits increased expression (as compared to wild type) of the heterologous protein.

2. A system as claimed in Claim 1 wherein said host is genomically altered by impairing the ability of the host to produce an aporepressor activator for said regulatable promoter.

3. A system as claimed in Claim 1 or Claim 2 wherein said regulatable promoter is the trp promoter.

4. A system as claimed in Claim 1 or Claim 3 when dependent upon Claim 1 wherein said alteration results in a less active form of the native encoded product expression of which is normally under control of said indigenous promoter.

5. A system as claimed in Claim 4 wherein said alteration is in the trpA gene and said host is a derivative of E. coli K12 strain MM294.

6. A system as claimed in Claim 5 which is E. coli MM294/A46PR9, obtainable from ATCC 53075, transformed with an expression vector containing the trp promoter.

7. The use of an expression system as defined in any one of Claims 1 to 6 in recombinant protein production.

8. E. coli MM2954/A46PR9, obtainable from ATCC 53075.

9. A process for producing a host for the production, with increased efficiency, of recombinant heterologous protein not involved in the biosynthesis of tryptophan, which process comprises:
modifying a host DNA sequence which is under the control of an indigenous regulatable promoter and encodes an enzyme responsible for the synthesis of aporepressor-activator so that the host DNA sequence has diminished ability to synthesise the aporepressor-activator,
and transforming the host with another DNA sequence containing a desired heterologous protein coding sequence in operable linkage to the regulatable promoter.

## Patentansprüche

1. Rekombinantes Expressionssystem für heterologe Proteine, die nicht an der Biosynthese von Tryptophan beteiligt sind, umfassend:
einen prokaryontischen Wirt mit einem Operon, das durch einen angeborenen regulierbaren Promotor kontrolliert wird, wobei der Wirt mit einer DNA-Sequenz transformiert ist, die eine gewünschte heterologe codierende Sequenz funktionell verknüpft mit dem regulierbaren Promotor umfaßt,
wobei das Operon eine Mutation in einer Sequenz eingebaut hat, die ein Enzym codiert, welches für die Synthese von Aporepressor-Aktivator verantwortlich ist, was zur verminderten Fähigkeit zur Synthese des Aporepressor-Aktivators führt, was die verstärkte Expression (verglichen mit dem Wildtyp) des heterologen Proteins erlaubt.

2. System nach Anspruch 1, wobei der Wirt genomisch verändert ist, indem die Fähigkeit des Wirts zur Produktion eines Aporepressor-Aktivators für den regulierbaren Promotor vermindert ist.

3. System nach Anspruch 1 oder 2, wobei der regulierbare Promotor der trp-Promotor ist.

4. System nach Anspruch 1 oder nach Anspruch 3, der abhängig ist von Anspruch 1, wobei die Änderung zu einer weniger aktiven Form des nativen codierten Produkts führt, dessen Expression normalerweise unter Kontrolle des angeborenen Promotors ist.

5. System nach Anspruch 4, wobei die Änderung im trpA-Gen liegt und der Wirt ein Derivat des E. coli K12-Stammes MM294 ist.

6. System nach Anspruch 5, nämlich E. coli MM294/A46PR9, erhältlich aus ATCC 53075, transformiert mit einem den trp-Promotor enthaltenden Expressionsvektor.

7. Verwendung eines Expressionssystems nach einem der Ansprüche 1 bis 6 für die Produktion eines rekombinanten Proteins.

8. E. coli MM2954/A46PR9, erhältlich von ATCC 53075.

9. Verfahren zur Herstellung eines Wirts für die mit erhöhter Wirksamkeit erfolgende Produktion des rekombinanten heterologen Proteins, das nicht an der Biosynthese von Tryptophan beteiligt ist, umfassend:
Modifikation einer Wirts-DNA-Sequenz, die unter der Kontrolle eines angeborenen regulierbaren Promotors ist und ein Enzym codiert, welches für die Synthese von Aporepressor-Aktivator verantwortlich ist, so daß die Wirts-DNA-Sequenz eine verminderte Fähigkeit zur Synthese des Aporepressor-Aktivators aufweist,
und Transformation des Wirts mit einer anderen DNA-Sequenz, die eine das gewünschte heterologe Protein codierende Sequenz in funktioneller Verknüpfung mit dem regulierbaren Promotor aufweist.

## Revendications

1. Système d'expression recombinant pour des protéines hétérologues qui ne participent pas à la biosynthèse de tryptophane qui comprend:
un hôte procaryote possédant un opéron contrôlé par un promoteur indigène régulable, lequel hôte est transformé avec une séquence d'ADN contenant une séquence codante hétérologue désirée, lié de manière opérationnelle au promoteur régulable,
caractérisé en ce que l'opéron contient une mutation dans une séquence codant pour une enzyme responsable de la synthèse de l'activateur de l'aporépresseur responsable de la diminution de l'aptitude à synthétiser l'activateur du répresseur ce qui permet une expression accrue (par rapport au type sauvage) de la protéine hétérologue.

2. Système selon la revendication 1 caractérisé en ce que ledit hôte est modifié au niveau génomique de manière à induire une perte de l'aptitude de l'hôte à produire un activateur de l'aporépresseur pour ledit promoteur régulable.

3. Système selon la revendication 1 ou 2 caractérisé en ce que ledit promoteur régulable est le promoteur trp.

4. Système selon la revendication 1 ou 3 si cette dernière dépend de la revendication 1, caractérisé en ce que ladite altération est responsable d'une forme moins active du produit natif codé dont l'expression est normalement sous le contrôle dudit promoteur indigène.

5. Système selon la revendication 4 caractérisé en ce que ladite altération se trouve au sein du gène de trp A et ledit hôte est un dérivé de la souche K12 MM294 de E. coli.

6. Système selon la revendication 5 qui est la souche Mm294/A46PR9 de E. coli, que l'on peut obtenir auprès de l'ATCC sous le numéro 53073, transformée par un vecteur d'expression contenant le promoteur trp.

7. Utilisation d'un système d'expression défini dans l'une des revendications 1 à 6 pour la production d'une protéine recombinante.

8. Souche Mm294/A46PR9 de E. coli, disponible auprès de l'ATCC sous le numéro 53073.

9. Procédé pour la production d'un hôte destiné à la production, avec une efficacité accrue, d'une protéine hétérologue recombinante qui ne participe pas à la biosynthèse du tryptophane, lequel procédé comprend:
la modification d'une séquence d'ADN de l'hôte qui est sous le contrôle d'un promoteur indigène régulable et code pour une enzyme responsable de la synthèse de l'activateur de l'aporépresseur de telle sorte que l'aptitude de la séquence d'ADN de l'hôte à synthétiser l'activateur de l'aporépresseur est réduite,
et la transformation de l'hôte avec une autre séquence d'ADN contenant une séquence codant pour la protéine hétérologue désirée liée de manière opérationnelle avec le promoteur régulable.
